(12) **EUROPEAN PATENT SPECIFICATION**

(45) Date of publication of patent specification: **16.09.92**

(21) Application number: **87307386.0**

(22) Date of filing: **20.08.87**

(51) Int. Cl.⁵: **C12N 15/00**, A61K 39/155, //C12Q1/68,C12Q1/70, C07K15/00

(54) **DNA Coding for rinderpest virus antigen, its preparation and use.**

(30) Priority: **29.08.86 JP 201765/86**
**26.03.87 JP 70413/87**

(43) Date of publication of application:
**02.03.88 Bulletin 88/09**

(45) Publication of the grant of the patent:
**16.09.92 Bulletin 92/38**

(84) Designated Contracting States:
**BE CH DE FR GB LI LU NL SE**

(56) References cited:

**CHEMICAL ABSTRACTS, vol. 102, no. 19, 13th May 1985, page 160, abstract no. 161472v, Columbus, Ohio, US; S.E.H. RUSSELL et al.: "cDNA cloning of the messenger RNAs of five genes of canine distemper virus", & J. GEN. VIROL. 1985, 66(3), 433-41**

(73) Proprietor: **Tonen Corporation**
**1-1 Hitotsubashi, 1-Chome Chiyoda-Ku Tokyo 100(JP)**

(72) Inventor: **Yamanouchi, Kazuya**
**3-8-7-111, Miyoshi-cho**
**Fuchu-shi Tokyo(JP)**
Inventor: **Yoshikawa, Yasuhiro**
**3-1-6, Shinmachi**
**Hoya-shi Tokyo(JP)**
Inventor: **Sugimoto, Masanobu**
**2-3-4-702, Tate**
**Shiki-shi Saitama(JP)**

(74) Representative: **Bizley, Richard Edward et al**
**BOULT, WADE & TENNANT 27 Furnival Street London EC4A 1PO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 106, no. 13, 30th March 1987, pages 171-172, abstract no. 97047p, Columbus, Ohio, US; C. GERALD et al.: "Measles virus hemagglutinin gene: cloning, complete nucleotide sequence analysis and expression in COS cells", & J. GEN. VIROL. 1986, 67(12), 2695-703

BIOLOGICAL ABSTRACTS, vol. 84, 1987, abstract no. 107714; K. TSUKIYAMA et al.: "Molecular cloning and sequence analysis of the rinderpest virus messenger RNA encoding the hemagglutinin protein", & VIROLOGY(US) 1987, vol. 160, no. 1, p. 48-54

**Description**

The present invention relates, inter alia, to DNA coding for antigen proteins of the rinderpest virus, a process for the production of that DNA, and a process for the production of the antigen protein using that DNA.

Rinderpest is often prevalent in many areas of the world, such as the Middle and Near East and Africa etc., and, therefore, there is intensive research into the development of vaccines for the rinderpest virus.

In the development of vaccines through genetic engineering, a process wherein a gene coding for an antigen protein of the vaccine is expressed in E. coli, yeast or animal cells to obtain the antigen protein, and processes wherein a gene coding for an antigen protein is integrated into a vaccinea virus to construct a recombinant virus for the production of a live vaccine have been suggested. To carry out such process, a gene coding for an antigen protein of the rinderpest virus must be cloned.

A rinderpest virus strain L that can be grown in Vero cells has been constructed (F. Kobune et al, Archives of Virology, 68, 271-177, (1981). Also, a rinderpest virus strain L (L 13) that can be grown in Vero cells has been developed (J. Gen. Virol. 67, 271, 1986).

The cloning of a cDNA coding for an F protein of the canine distemper virus has been disclosed by S.E.H. Russell, J. Gen. Virol. 66, 435-441, 1985.

Nevertheless, a gene coding for an antigen protein of the rinderpest virus has not hitherto been cloned.

Accordingly, the present invention provides cloned genes coding for an antigen proteins of the rinderpest virus and describes process for the production thereof,

and a process for the production of an antigen protein which is promising as an active ingredient for vaccines against the rinderpest virus.

More particularly, the present invention provides DNA coding for the entirety or a part of an antigen protein of the rinderpest virus wherein said antigen protein is:

a) a hemagglutinin protein encoded by the nucleotide sequence:

ATGTCCTCCC CAAGAGACAG GGTCAATGCC
TTCTACAAAG ACAACCTCCA ATTTAAGAAC ACTCGAGTGG TTCTTAATAA
AGAGCAGCTC CTGATAGAAA GGCCTTACAT GTTGCTGGCG GTGCTGTTTG
TTATGTTCCT GAGCCTAGTG GGGCTGTTGG CCATTGCAGG TATCAGACTC
CACCGAGCTG CTGTCAACAC AGCAGAGATC AACAGTGGTC TGACGACAAG
CATTGATATT ACCAAGTCTA TTGAGTACCA GGTCAAGGAC GTCTTAACTC
CCCTCTTCAA AATAATTGGA GATGAGGTCG GGCTGAGGAC ACCTCAGAGA
TTCACAGATC TGACTAAATT CATATCAGAC AAGATTAAGT TCCTTAACCC
TGATAAAGAG TACGACTTCA GGGATATTAA CTGGTGCATC AGTCCCCCAG
AGAGAATCAA GATTAATTAT GATCAGTATT GTGCTCACAC AGCTGCTGAG
GAGCTGATAA CTATGCTGGT CAATTCGTCT CTGGCAGGTA CTTCGGTACT
ACCGACATCA TTAGTCAACT TGGGGAGGAG CTGTACCGGG TCCACAACGA
CTAAAGGTCA ATTCTCTAAC ATGTCATTGG CTCTTTCAGG GATATACTCA
GGTCGTGGCT ACAATATTTC ATCCATGATA ACAATCACTG AGAAAGGCAT
GTACGGAAGC ACTTATCTAG TCGGGAAACA TAATCAGGGA GCCAGGAGGC
CAAGCACTGC TTGGCAACGG GATTACCGAG TCTTTGAAGT AGGCATAATT
AGAGAACTAG GACTGGGCAC ACCAGTGTTT CATATGACAA ACTACCTGGA
GCTCCCAAGA CAGCCGGAAT TGGAGATCTG CATGCTAGCT CTAGGAGAGT
TCAAATTAGC TGCCCTCTGC TTAGCTGATA ACTCTGTCGC ACTGCATTAC
GGGGGGGTTAA GGGACGACCA CAAGATCAGG TTTGTCAAAC TGGGAGTATG
GCCATCACCA GCCGACTCAG ACACCCTGGC CACTCTTTCA GCAGTAGATC
CGACCTTGGA TGGGCTCTAT ATCACAACTC ATAGGGGAAT CATAGCTGCA

```
GGGAAGGCCG TATGGGTCGT CCCTGTGACG AGAACAGATG ACCAAAGGAA
AATGGGACAG TGCCGCCGAG AGGCTTGTCG AGAGAAACCA CCACCTTTCT
GTAACAGTAC AGATTGGGAG CCATTAGAGG CCGGCCGTAT ACCGGCATAT
GGAATACTAA CTATCAGGCT GGGGCTGGCT GATAAGCTGA AATTGACCAT
AATTTCAGAA TTTGGTCCCT TGATCACACA TGACTCAGGG ATGGACTTAT
ACACCCCACT TGACGGTAAT GAGTACTGGC TGACTATTCC TCCATTGCAG
AATTCAGCTT TAGGAACGGT GAACACCCTA GTTTTAGAGC CCAGTCTCAA
AATTAGTCCT AACATCCTTA CTCTCCCCAT CAGGTCGGGG GGAGGTGACT
GTTACACTCC CACTTACCTG TCAGACCTGG CCGATGATGA TGTTAAACTG
AGCTCCAATC TTGTAATCCT CCCGAGTAGA AACCTCCAAT ATGTGTCAGC
AACCTACGAC ACCTCTAGAG TTGAGCATGC CATTGTATAC TATATCTATA
GCGCCGGGCG ACTATCATCG TATTACTACC CTGTTAAGTT GCCCATAAAG
GGAGATCCTG TCAGCCTGCA GATAGGATGC TTCCCTTGGG GCCTCAAGCT
ATGGTGCCAT CATTTCTGCT CTGTTATAGA TTCAGGAACT CGCAAGCAGG
TCACCCATAC AGGGGCAGTA GGGATTGAGA TCACTTGCAA TAGCAGATAG;
```

or a DNA sequence hybridizable with the aforesaid sequence and encoding part at least of a rinderpest virus antigenic polypeptide or a DNA sequence as aforesaid modified by the deletion or addition of one or more codons or the modification of one or more codons but still encoding a rinderpest virus antigenic polypeptide or

b) a fusion protein encoded by the nucleotide sequence:

```
           ATGG GGATCTTATT TGCTGCCCTG CTAGCAATGA
CTAACCCACA CTTAGCTACT GGCCAGATCC ACTGGGGCAA CCTTTCAAAA
ATTGGACTCG TAGGGACAGG TAGTGCCAGT TATAAGGTGA TGACCCAATC
GAGTCACCAG TCACTAGTTA TAAAGCTGAT GCCCAATATC ACTGCTATAG
ACAATTGCAC CAAAACAGAG ATTATGGAGT ACAAGAGGCT GTTGGGGACA
GTGCTTAAGC CTATCAGGGA AGCTCTTAAC GCTATTACTA GAACATAAA
ACCAATCCAG AGTTCCACTA CCAGTAGGAG ACACAAGAGG TTCGCGGGAG
TCGTTTTGGC TGGAGCCGCT CTCGGGGTTG CAACCGCAGC CCAGATTACA
GCAGGGATTG CTCTTCATCA GACAATGATG AATTCCCAAG CTATCGAAAG
TCTTAAGGCA AGTCTGGAAA CAACCAATCA AGCAATTGAG GAAATACGAC
AAGCGGGTCA AGAAATGGTC CTAGCGGTTC AGGGTGTCCA AGATTACATC
AACAATGAAC TGGTGCCTGC GATGGGCCAG CTATCGTGTG AAATTGTGGG
TCAAAAGCTA GGGCTGAAAC TGCTTAGATA TTACACTGAA ATCTTGTCAT
```

```
TATTTGGACC CAGCCTCAGG GACCCGGTCT CGGCTGAGCT TTCTATTCAG

GCCTTAAGTT ACGCCTTGGG TGGGGACATT AATAAAATAC TAGAAAAGCT

TGGGTATAGC CCCACTGATC TCCTTGCTAT ACTGGAAAGT AAGGGTATCA

AGGCTAAGAT AACCTATGTG GATATCCAAA GTTACTTCAT TGTGCTCAGC

ATAGCTTATC CGTCACTCTC CGAAATTAAG GGGGTGATAG TGCACCGTCT

GGAGACTGTC TCTTACAATA TAGGATCGCA GGAGTGGTAC ACCACAGTAC

CGCGGTATGT GGCAACCCAG GGTTACCTCA TTTCAAATTT CGATGACACA

CCCTGTGCAT TCACTCCGGA GGGCACTATC TGCAGTCAAA ATGCAATATA

CCCGATGAGC CCATTGCTCC AAGAATGTTT CCGTGGGTCA ACCAGGTCAT

GTGCCCGTAC ACTGGTTTTG GGGTCCATAG GGAATAGGTT TATACTATCT

AAGGGGAACC TCATTGGCAA CTGTGCCTCT ATTTTGTGCA AGTGCTACAC

CACTGGCTCA ATAATAAGTC AAGACCCTGA CAAGATCCTA ACATATATAG

CTGCAGACCA ATGCCCCGTC GTAGAAGTAG GCGGTGTAAC CATCCAGGTC

GGGAGCAGGG AGTACTCCGA TGCAGTGTAC CTGCATGAAA TAGATCTTGG

CCCACCAATA TCGCTAGAGA AGCTGGATGT GGGGACTAAC CTCTGGAATG

CAGTGACAAA ATTGGAGAAA GCCAAGGATC TACTAGATTC ATCTGATCTG

ATCCTGGAAA ACATCAAGGG TGTTTCAGTC ACGAACACAG GTTACATTOT

AGTCGGAGTC GGGTTGATTG CAGTGGTGGG GATCCTCATT ATTACCTGTT

GTTGTAAGAA GCGCAGGACT GACAACAAAG TCTCCACTAT GGTCTTGAAC

CCGGGTCTTA GACCAGATCT TACTGGTACA TCTAAATCCT ACGTACGGTC

GTTGTAG;
```

or a DNA sequence hybridizable with the aforesaid sequence and encoding part at least of a rinderpest virus antigenic polypeptide or a DNA sequence as aforesaid modified by the deletion or addition of one or more codons or the modification of one or more codons but still encoding a rinderpest virus antigenic polypeptide.

The present specification also describes a process for the production of the above-mentioned DNA comprising the steps of:

preparing an mRNA from the rinderpest virus;
preparing a cDNA library from the mRNA;
selecting a cDNA coding for the target protein from the cDNA library; and
cloning the selected cDNA in a cloning vector.

The present specification also describes a process for the production of the above-mentioned protein comprising the steps of:

transfecting a vector containing the DNA coding for the target protein into animal cells;
culturing the animal cells to produce the antigen protein; and
recovering the target protein from the cell culture.

In the drawings:-

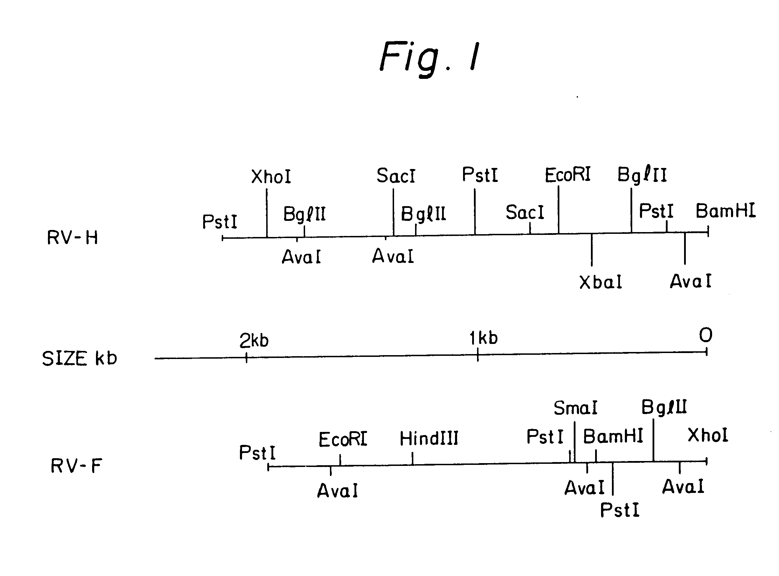

Figure 1 represents a restriction enzyme cleavage map of a cDNA insert RV-H in a plasmid pDH-RVH and a restriction enzyme cleavage map of a cDNA insert RV-F in a plasmid pDH-RVF;

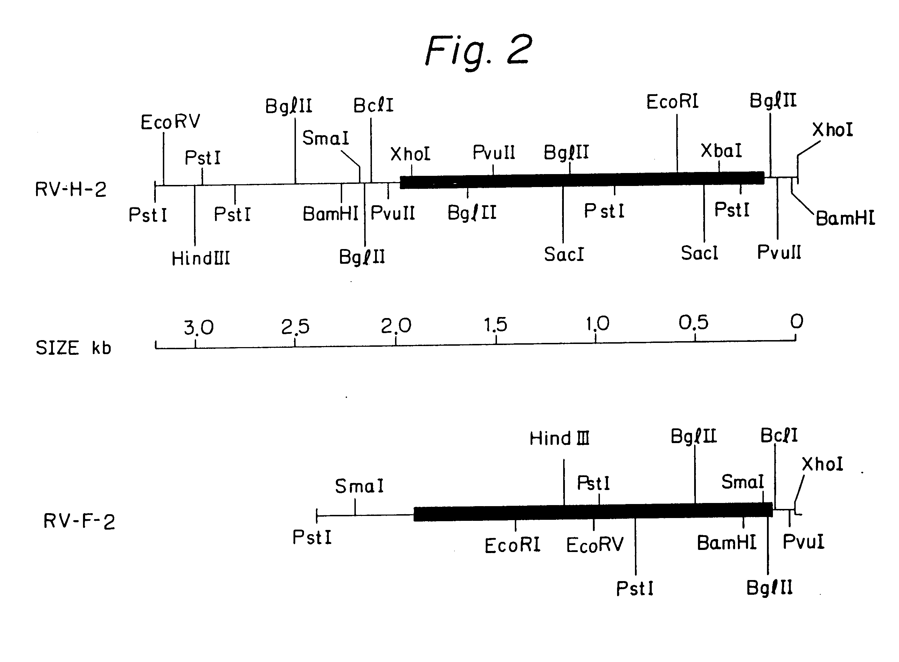

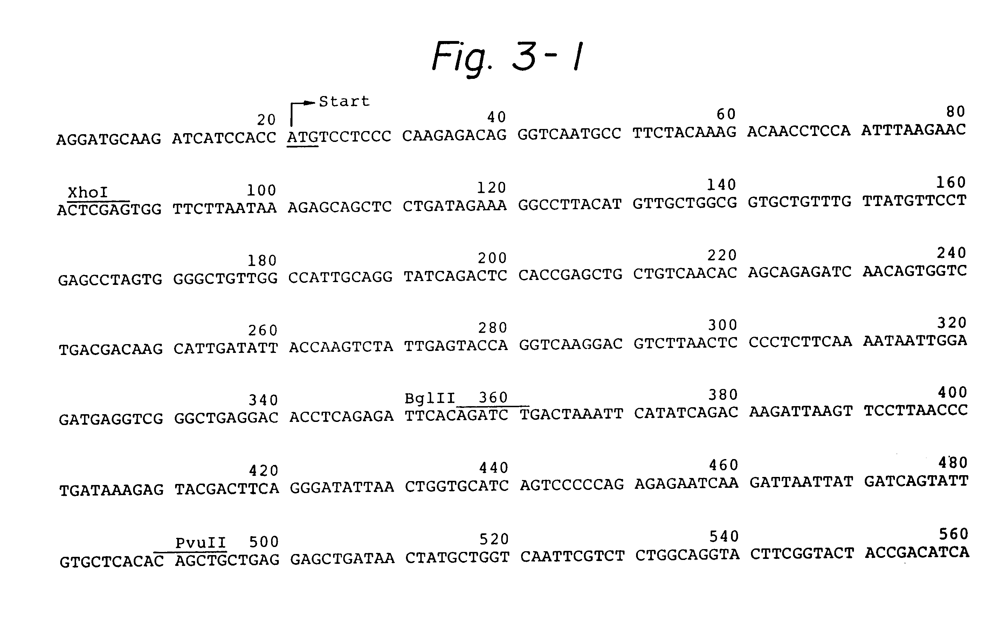

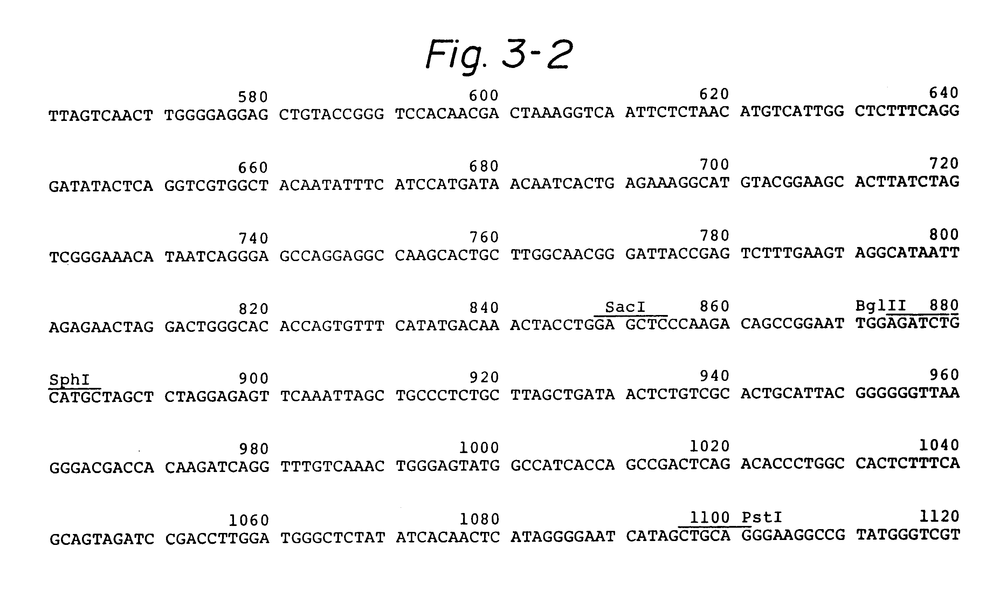

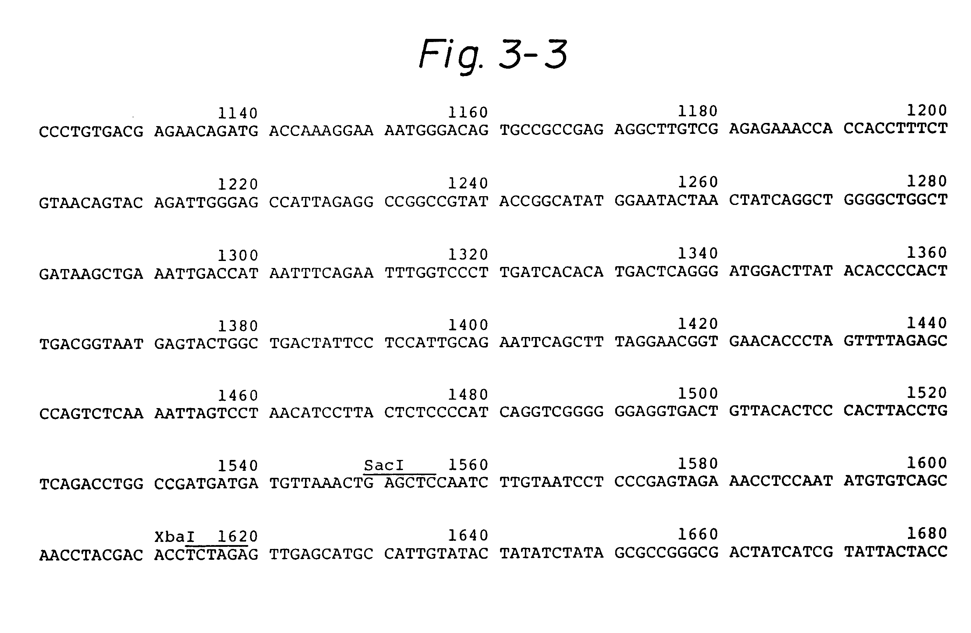

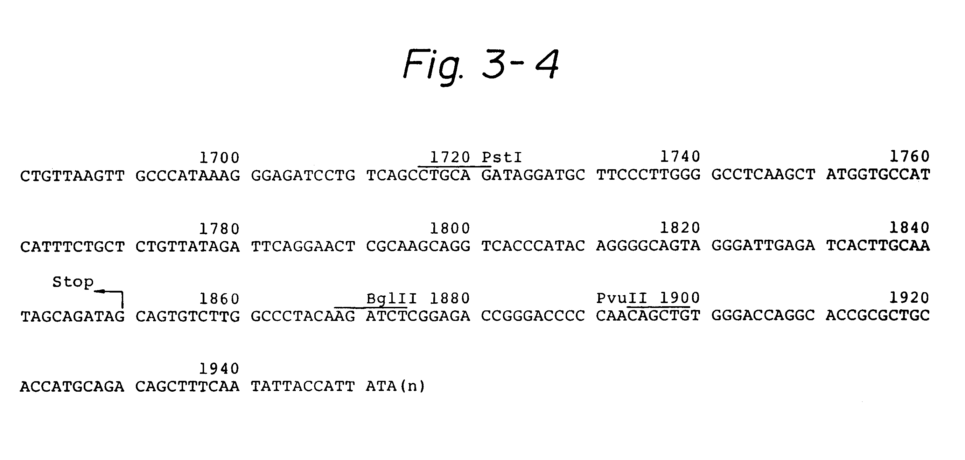

Fig. 2 represents a restriction enzyme cleavage map of a cDNA insert RV-H-2 in a plasmid pDH-RVH-2 and a restriction enzyme cleavage map of a cDNA insert RV-F-2 in a plasmid pDH-RVF-2, wherein the thick line in the RV-H-2 represents a portion corresponding to a nucleotide sequence coding for a hemagglutinin protein (H protein) of the rinderpest virus shown in Fig. 3; and,

Figs. 3-1 to 3-4 represent a nucleotide sequence containing a hemagglutinin gene of the rinderpest virus, wherein, in the sequence, ATG at the 21st to 23rd position is a translation start codon and TAG at the 1848th to 1850th position is a translation stop codon, and a reading frame flanked by these codons

6

(represented by a thick line in Fig. 2, RV-H-2) shows the codes for a hemagglutinin protein.

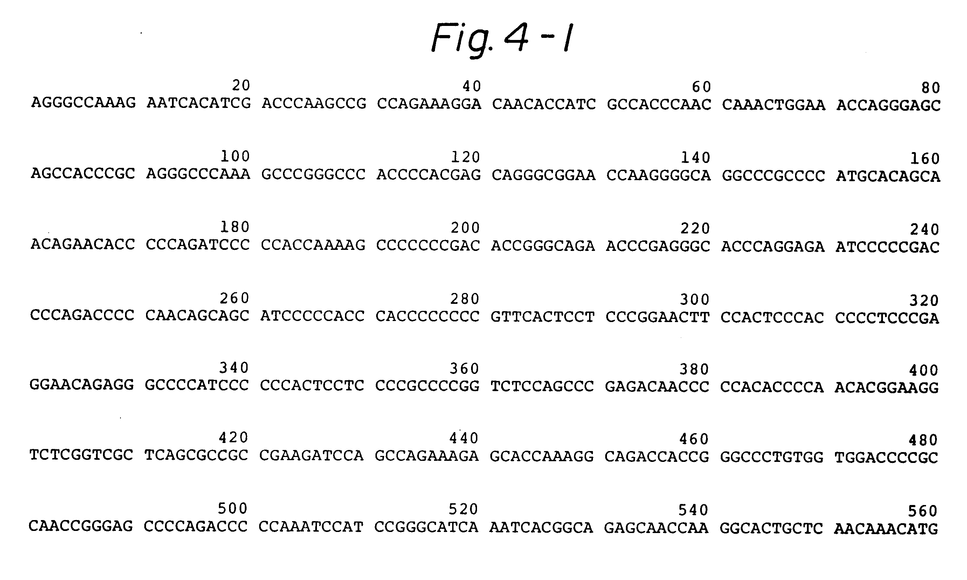

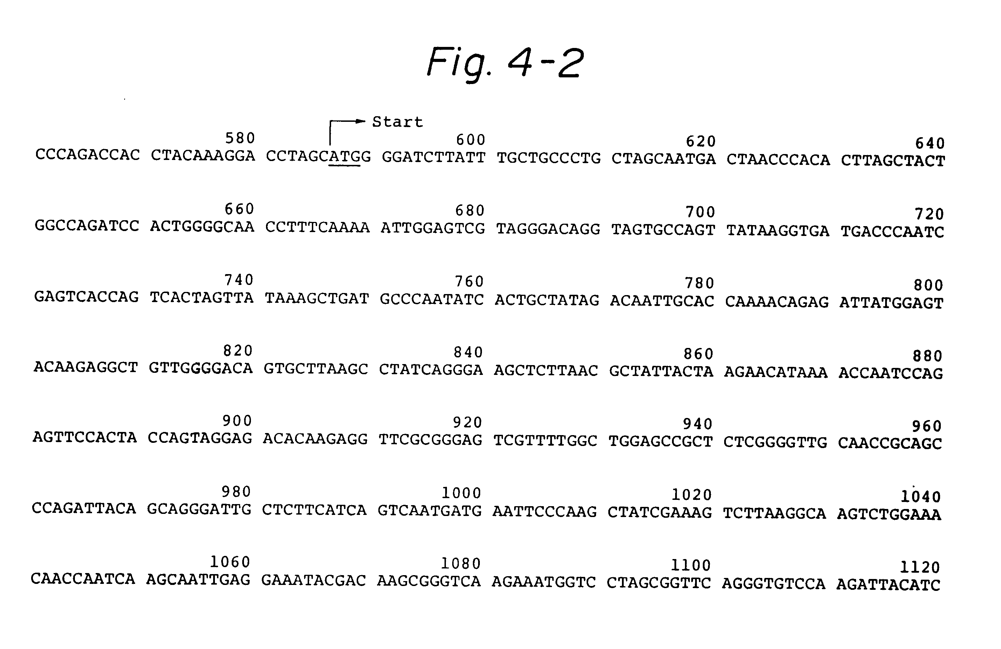

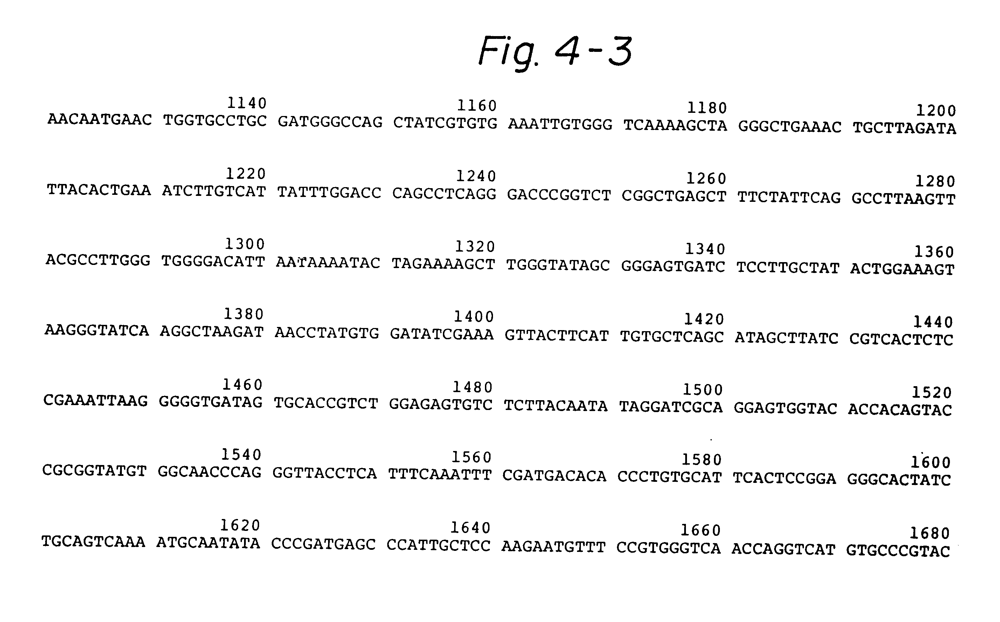

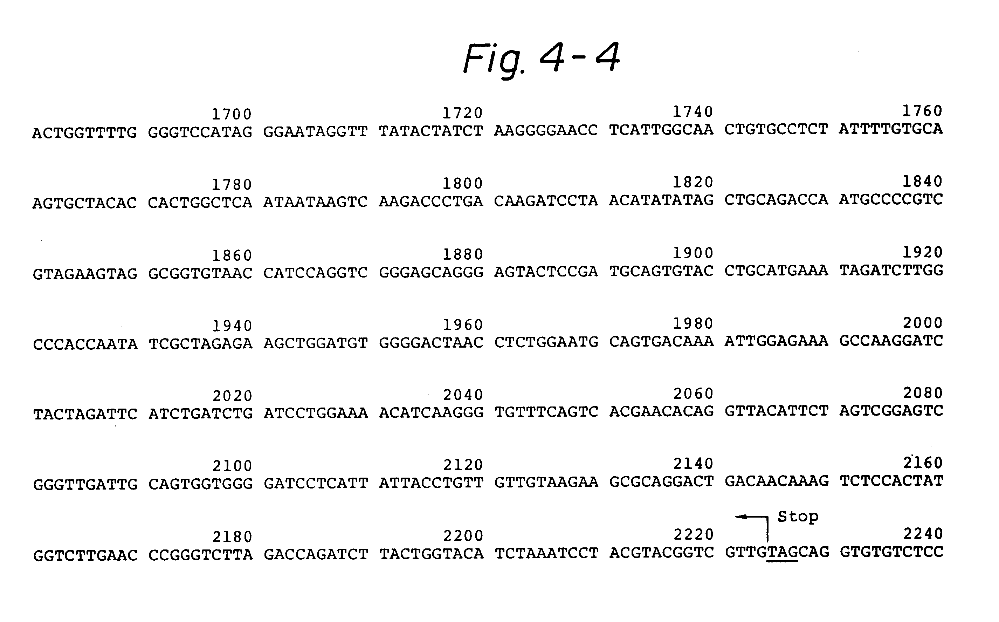

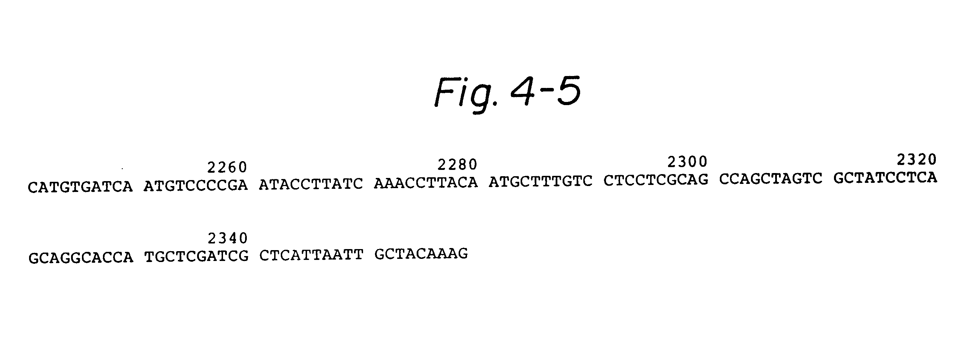

Figs. 4-1 to 4-5 represent a nucleotide sequence containing a fusion protein gene of the rinderpest virus, wherein, in the sequence, ATG at the 587th to 589th position is a translation start codon and TAG at the 2225th to 2227th position is a translation stop codon, and a reading frame flanked by these codons (represented by a thick line in Fig. 2, RV-F-2) shows the codes for a fusion protein.

A. Cloning of genes

Since it is believed that proteins effective as antigens to the rinderpest virus are the hemagglutinin protein (H protein) and the fusion protein (F protein), according to the present invention, the genes coding for these proteins were cloned.

(1) Extraction of mRNA

The rinderpest virus L strain that can be grown in Vero cells (rinderpest virus L 13 strain) was cultured, mRNA was extracted from the culture, and the cDNA was prepared from the mRNA according to the Okayama-Berg method (Okayama and Berg, Mol. Cell Boil., 2, 161-170, 1983; and Mol. Cell Biol., 3, 280-289, 1983).

More specifically, the rinderpest virus L 13 was used as the origin of the mRNA. The L 13 strain was obtained by fusing a rabbit lymphocyte infected with a rabbit-adapted passaged strain of the rinderpest virus (L-lapinized strain) and F-Vero cells, under polyethylene glycol, and passaging 15 generations. Sub-confluent F-Vero cells in eight Petri dishes each having a diameter of 6 cm were inoculated with the L 13 strain at an m.o.i. of 1. The culture was passaged on the next day, and 24 hours after the passage, 20 μg/ml of actinomycin D was added to the culture to inhibit synthesis of cellular mRNA and incubation was continued for an additional 5 hours. The recovered cells were centrifuged in 6 M of guanidinumisothiocyanate (GTC), 5 mM of sodium citrate and 0.5% of Sarkosyl - 5.7 M of $CsCl_3$ and 0.1 M of EDTA at 35,000 rpm (150,000 XG) for more than 12 hours, according to the guanidinum-cesium chloride method, to recover the RNA (T. Maniatis et al., Molecular Cloning, p 196, 1982).

The thus-recovered RNA was the applied to an oligo (dT) cellulose column, and the adsorbed RNA was eluted by 10 mM Tris-HCl (pH 7.5) buffer containing 1 mM EDTA and 0.05% SDS to obtain a poly $A^{(+)}$ RNA according to a known procedure (T. Maniatis et al., Molecular Cloning, p 197, 1982).

(2) Preparation of probes

According to the present invention, to confirm the presence of a target mRNA in the above-prepared poly $A^{(+)}$ RNA, and to screen a cDNA library as described hereinafter in detail, an H gene of a subacute sclerosing panencephalitis (SSPE) virus was used as a probe (designated as H gene probe or SSPE-H-cDNA probe) to detect and screen the gene coding for the H protein (H gene) of the rinderpest virus; and the F gene of the canine distemper virus (CDV) was used as a probe (designated as F gene probe or CDV-F-cDNA probe) to detect and screen the gene coding for an F protein (F gene) of the rinderpest virus. These genes were selected as probes because the above-mentioned SSPE virus and CDV, as well as the rinderpest virus, belong to the common genus Morbillivirus and these viruses are closely related to each other, and therefore, it is considered that the nucleotide sequences thereof will have a high homology.

The above-mentioned probes were prepared by obtaining an SSPE virus DNA and CDV DNA and labeling these DNA with a radioisotope $^{35}$S (T. Maniatis et at., Molecular Cloning, p 109, 1982).

More specifically, a cDNA library was made from SSPE infected cells, and SSPE H cDNA was selected by the use of measles virus H probe (Billeter et al. 1984, Virology 145 195). (DV H DNA was made according to Russel et al. (1985) (J. Gen. Virol, 66 433.). Each DNA thus-prepared was incubated with three unlabeled deoxyribonucleotides dATP, dGTP and dTTP, and a $^{35}$S-labeled deoxyribonucleotide $^{35}$S-dCTP-γS in the presence of E. coli DNA polymerase I in a Tris-HCl (pH 7.8) buffer. The labeled DNA was then separated from unreacted $^{35}$S-dCTP-γS by a Sephadex G25 column.

(3) Detection of H protein mRNA and F protein mRNA in poly $A^{(+)}$ RNA

The poly $A^{(+)}$ RNA prepared as described above was denatured with glyoxal or formamide, and separated by Agarose gel electrophoresis, and the separated mRNAs were subjected to hybridization with the above-mentioned SSPE-H-cDNA probe or CDV-F-cDNA probe according to the conventional Northern hybridization method. As a result, a 1.96 kb mRNA which hybridizes with the SSPE-H-cDNA probe and a 2.2 kb mRNA which hybridizes with the CDV-F-cDNA were detected. This result suggests that the poly $A^{(+)}$ RNA preparation includes an mRNA coding for the H protein of the rinderpest virus and an mRNA coding for the F protein of the rinderpest virus.

(4) Preparation of cDNA library

A cDNA library was prepared from the above-mentioned poly $A^{(+)}$ RNA preparation according to the Okayama-Berg method (Okayama H. and P. Berg, Mol. Cell Biol., 2, 161, 1982; and Mol. Cell Biol., 3, 280, 1983). As a vector, an oligo dT-tailed (T = 17) plasmid primer (pcDV-1) and an oligo dG-tailed (G = 12) pL-1 Hind III linker were used.

More specifically, 8µg of the above-mentioned poly $A^{(+)}$ RNA, 2 µg of the dT-tailed plasmid primer (pcDV-1), 2 mM of four dNTPs including $^{35}$S-dCTP, and 20.6 units of a reverse transcriptase (RTase) were incubated in 40 µl of a reaction mixture containing 100 mM of Tris-HCl (pH 8.3), 10 mM of $MgCl_2$, 140 mM of KCl, 2 mM of methylmercury hydroxide, 20 mM of mercaptoethanol, and 1 mM of RNase inhibitor at 42°C for 60 minutes, to form a cDNA-plasmid. To the reaction mixture, 3.5 mM of dCTP and 30 units of terminal transferase were added, resulting in the formation of 73.5 µl total volume of the reaction mixture. The reaction mixture was subjected to reaction at 37°C for 3 minutes, and 2.5 mM of EDTA containing 0.5% (final concentration) of SDS was added to the reaction mixture to terminate the reaction. The reaction mixture was extracted with chloroform and phenol, and the DNA was then precipitated with ethanol.

The thus-prepared cDNA-plasmid was digested with 20 units of Hind III in 40 µl of a buffer containing 10 mM of Tris-HCl (pH 7.5), 50 mM of NaCl, 10 mM of $MgCl_2$ and 1 mM of dithiothreitol, at 37°C for 2 hours. Then, 120 mg of the digested cDNA-plasmid and 35 µg of the PL-1 linker were incubated in 40 µl of a reaction mixture containining 0.1 M NaCl, 10 mM Tris-HCl (pH 7.8) and 1 mM EDTA, first at 65°C for 5 minutes and that of 42°C for 60 minutes, and the reaction mixture was then cooled to a room temperature to allow annealing. After the reaction mixture was cooled to 0°C, 0.3 µg of E. coli DNA ligase was then added to the reaction mixture, and the reaction mixture was incubated at 12°C overnight.

Then, 200 mg of E. coli DNA ligase, 0.450 units of RNase H and 0.8 units of DNA polymerase were added to the reaction mixture to make the total volume of the mixture 48 µl, and the temperature of the reaction mixture was then increased stepwise to 12°C, 15°, 20°C, and to 25°C, for 30 minutes at each temperature, resulting in digestion of an mRNA region, formation of a double-stranded cDNA and ligation of the resulting cDNA, to complete the construction of plasmids containing the cDNA.

The thus-obtained plasmids were used to transform Escherichia coli DH-5 and E. coli MC1061, and a cDNA library comprising 7,700 clones and a cDNA library comprising 7,300 clones (total 15,000 clones) were obtained from E. coli DN-5 and E. coli MC1061, respectively.

(5) Screening of cDNA Library

From among the above-mentioned 15,000 clones, 109 clones were screened by the colony hybridization method. As a result, a clone hybridizing with the SSPE-H-cDNA probe and a clone hybridizing with CDV-F-cDNA were obtained. These clones were designated as Escherichia coli DH-RVH and Escherichia coli DH-RVF respectively; and plasmids in these E. coli cells were designated as pDH-RVH and pDH-RVH. The size of cDNA insert (designated as RV-H) in the plasmid pDH-RVH was about 2.2 kb, and the size of the cDNA insert (designated as RV-F) in the plasmid pDH-RVF was about 1.6 kb. It was considered that these cDNA inserts contained approximately the entire coding regions for the H protein and F protein of the rinderpest virus, respectively.

The above-mentioned Escherichia coli DH-RVH was deposited with the Fermentation Research Institute Agency of Industrial Science and Technology (FRI), Higashi 1-1-3, Yatabe-cho, Tsukuba-gun, Ibaraki-ken, Japan, as FERM BP-1165 under the Budapest treaty on August 29, 1986. The above-mentioned Escherichia coli DH-RVF was deposited with the FRI as FERM BP-1164 under the Budapest treaty on August 29, 1986.

By repeating the same procedure as described above, a clone hybridizing with the SSPE-H-cDNA probe and a clone hybridizing with the CDV-F-cDNA were obtained. These clones were designated as Escherichia coli DH-RVH-2 and Escherichia coli DH-RVF-2, respectively; and plasmids in these E. coli cells were designated as pDH-RVH-2 and pDH-RVF-2, respectively. The size of the cDNA insert (designated as RV-H-2) in the plasmid pDH-RVH-2 was about 3.2 kb, and the size of the cDNA insert (designated as RV-F-2) in the plasmid pDH-RVF-2 was about 2.4 kb. The RV-H-2 contained an entire coding region for the H protein of the rinderpest virus, and the RV-F-2 is considered to contain an entire coding region for the F protein of the rinderpest virus.

The above-mentioned Escherichia coli DH-RVH-2 was deposited with the FRI as FERM BP-1319 under the Budapest treaty on March 24, 1987. The above-mentioned Escherichia coli DH-RVF-2 was deposited with the FRI as FERM BP-1318 under the Budapest treaty on March 24, 1987.

(6) Restriction enzyme cleavage map

The restriction enzyme cleavage sites of the above-mentioned cDNA inserts were analyzed to determine restriction enzyme cleavage maps, according to a conventional procedure. Figure 1 represents the restriction enzyme cleavage maps for the cDNA inserts RV-H and RV-F, and Fig. 2 represents the restriction enzyme cleavage maps for the cDNA inserts RV-H-2 and RV-F-2.

(7) Determination of nucleotide sequences of cDNAs coding for H protein and F protein

The plasmids pDH-RVH and pDH-RVH-2 were digested with restriction enzymes Bam HI, Pvu II, Pst I, Eco RI, Hind III, and Sma I, and DNA fragments of several hundred base pairs in length were separated. Each DNA fragment was inserted in to a polylinker site of a phage M13 DNA, and the nucleotide sequence was determined according to the Sanger dideoxy chain termination method. The result is shown in Figs. 3-1 to 3-4.

According to a similar procedure, the nucleotide sequence coding for F protein was determined. The result is shown in Figs. 4-1 to 4-5.

(8) Expression of gene

COS7 cells were cultured in a cell culture chamber in 1 ml of Eagl's minimum essential medium containing 10% felal calf serum at 37°C for 24 hours. The DNA of the plasmid pDH-RVH-2 was coprecipitated with calcium phosphate, and 1 µg of the precipitated DNA was transfected in to the cultured COS7 cells. The cells were then treated with a DMEM medium containing 10% dimethylsulfoxide and 5% fetal calf serum. fresh medium was added to the cells, and culturing was carried out at 37°C for two to three days. The cells were then fixed with acetone, and were observed by an indirect fluorescent antibody technique using an antibody specific to the rubeola virus. In this technique, an antigen H reactive with the antibody was observed on the cells. In same manner, it was confirmed that the pDH-RVF and pDH-RVF2 expressed an antigen F.

The present cDNA coding for the hemagglutinin protein of the rinderpest virus, and the cDNA coding for the fusion protein of the rinderpest virus are useful as gene starting materials in a process for the construction of a recombinant virus by inserting them into, for example, a vaccinia virus, or in a process for the production of antigen proteins by expressing the cDNA in E. coli, yeast or animal cells.

Moreover, the present cDNA and fragments thereof are useful as hybridization probes for screening genes of different origin but containing a similar nucleotide sequence, such as cDNA of different origin. Moreover the present cDNA and fragments thereof are useful as components of a kit for the detection of the rinderpest virus and related viruses.

It should be noted that the procedures disclosed in the present specification can be applied to the cloning of other cDNAs coding for an antigen protein or a part thereof of the rinderpest virus. Such cDNA's are also covered by the present invention.

It will also be appreciated that the ambit of the invention is not limited to the precise DNA sequences disclosed herein. For obvious reasons of, inter alia, codon degeneracy and the uncertainty of paratopic/epitopic site definition, the invention also includes, in addition to the precise sequences, DNA sequences hybridizable with the aforesaid sequences and encoding part at least of a rinderpest virus antigenic polypetide or DNA sequences as aforesaid modified by the deletion or addition of one or more codons or the modification of one or more codons but still encoding a rinderpest virus antigenic polypeptide.

## Claims

1. DNA coding for the entirety or part of an antigen protein of a rinderpest virus wherein said antigen protein is:

   a) A hemagglutinin protein encoded by the nucleotide sequence:

```
                    ATGTCCTCCC CAAGAGACAG GGTCAATGCC
          TTCTACAAAG ACAACCTCCA ATTTAAGAAC ACTCGAGTGG TTCTTAATAA
          AGAGCAGCTC CTGATAGAAA GGCCTTACAT GTTGCTGGCG GTGCTGTTTG
          TTATGTTCCT GAGCCTAGTG GGGCTGTTGG CCATTGCAGG TATCAGACTC
          CACCGAGCTG CTGTCAACAC AGCAGAGATC AACAGTGGTC TGACGACAAG
          CATTGATATT ACCAAGTCTA TTGAGTACCA GGTCAAGGAC GTCTTAACTC
          CCCTCTTCAA AATAATTGGA GATGAGGTCG GGCTGAGGAC ACCTCAGAGA
          TTCACAGATC TGACTAAATT CATATCAGAC AAGATTAAGT TCCTTAACCC
          TGATAAAGAG TACGACTTCA GGGATATTAA CTGGTGCATC AGTCCCCCAG
          AGAGAATCAA GATTAATTAT GATCAGTATT GTGCTCACAC AGCTGCTGAG
          GAGCTGATAA CTATGCTGGT CAATTCGTCT CTGGCAGGTA CTTCGGTACT
          ACCGACATCA TTAGTCAACT TGGGGAGGAG CTGTACCGGG TCCACAACGA
          CTAAAGGTCA ATTCTCTAAC ATGTCATTGG CTCTTTCAGG GATATACTCA
          GGTCGTGGCT ACAATATTTC ATCCATGATA ACAATCACTG AGAAAGGCAT
          GTACGGAAGC ACTTATCTAG TCGGGAAACA TAATCAGGGA GCCAGGAGGC
          CAAGCACTGC TTGGCAACGG GATTACCGAG TCTTTGAAGT AGGCATAATT
          AGAGAACTAG GACTGGGCAC ACCAGTGTTT CATATGACAA ACTACCTGGA
          GCTCCCAAGA CAGCCGGAAT TGGAGATCTG CATGCTAGCT CTAGGAGAGT
          TCAAATTAGC TGCCCTCTGC TTAGCTGATA ACTCTGTCGC ACTGCATTAC
          GGGGGGTTAA GGGACGACCA CAAGATCAGG TTTGTCAAAC TGGGAGTATG
          GCCATCACCA GCCGACTCAG ACACCCTGGC CACTCTTTCA GCAGTAGATC
          CGACCTTGGA TGGGCTCTAT ATCACAACTC ATAGGGAAT CATAGCTGCA
```

```
GGGAAGGCCG TATGGGTCGT CCCTGTGACG AGAACAGATG ACCAAAGGAA
AATGGGACAG TGCCGCCGAG AGGCTTGTCG AGAGAAACCA CCACCTTTCT
GTAACAGTAC AGATTGGGAG CCATTAGAGG CCGGCCGTAT ACCGGCATAT
GGAATACTAA CTATCAGGCT GGGGCTGGCT GATAAGCTGA AATTGACCAT
AATTTCAGAA TTTGGTCCCT TGATCACACA TGACTCAGGG ATGGACTTAT
ACACCCCACT TGACGGTAAT GAGTACTGGC TGACTATTCC TCCATTGCAG
AATTCAGCTT TAGGAACGGT GAACACCCTA GTTTTAGAGC CCAGTCTCAA
AATTAGTCCT AACATCCTTA CTCTCCCCAT CAGGTCGGGG GGAGGTGACT
GTTACACTCC CACTTACCTG TCAGACCTGG CCGATGATGA TGTTAAACTG
AGCTCCAATC TTGTAATCCT CCCGAGTAGA AACCTCCAAT ATGTGTCAGC
AACCTACGAC ACCTCTAGAG TTGAGCATGC CATTGTATAC TATATCTATA
GCGCCGGGCG ACTATCATCG TATTACTACC CTGTTAAGTT GCCCATAAAG
GGAGATCCTG TCAGCCTGCA GATAGGATGC TTCCCTTGGG GCCTCAAGCT
ATGGTGCCAT CATTTCTGCT CTGTTATAGA TTCAGGAACT CGCAAGCAGG
TCACCCATAC AGGGGCAGTA GGGATTGAGA TCACTTGCAA TAGCAGATAG;
```

or a DNA sequence hybridizable with the aforesaid sequence and encoding part at least of a rinderpest virus antigenic polypeptide or a DNA sequence as aforesaid modified by the deletion or addition of one or more codons or the modification of one or more codons but still encoding a rinderpest virus antigenic polypeptide or

b) a fusion protein encoded by the nucleotide sequence:

```
          ATGG GGATCTTATT TGCTGCCCTG CTAGCAATGA
CTAACCCACA CTTAGCTACT GGCCAGATCC ACTGGGGCAA CCTTTCAAAA
ATTGGACTCG TAGGGACAGG TAGTGCCAGT TATAAGGTGA TGACCCAATC
GAGTCACCAG TCACTAGTTA TAAAGCTGAT GCCCAATATC ACTGCTATAG
ACAATTGCAC CAAAACAGAG ATTATGGAGT ACAAGAGGCT GTTGGGGACA
GTGCTTAAGC CTATCAGGGA AGCTCTTAAC GCTATTACTA AGAACATAAA
ACCAATCCAG AGTTCCACTA CCAGTAGGAG ACACAAGAGG TTCGCGGGAG
TCGTTTTGGC TGGAGCCGCT CTCGGGGTTG CAACCGCAGC CCAGATTACA
GCAGGGATTG CTCTTCATCA GACAATGATG AATTCCCAAG CTATCGAAAG
TCTTAAGGCA AGTCTGGAAA CAACCAATCA AGCAATTGAG GAAATACGAC
AAGCGGGTCA AGAAATGGTC CTAGCGGTTC AGGGTGTCCA AGATTACATC
AACAATGAAC TGGTGCCTGC GATGGGCCAG CTATCGTGTG AAATTGTGGG
TCAAAAGCTA GGGCTGAAAC TGCTTAGATA TTACACTGAA ATCTTGTCAT
```

EP 0 257 994 B1

```
TATTTGGACC CAGCCTCAGG GACCCGGTCT CGGCTGAGCT TTCTATTCAG

GCCTTAAGTT ACGCCTTGGG TGGGGACATT AATAAAATAC TAGAAAAGCT

TGGGTATAGC CCCACTGATC TCCTTGCTAT ACTGGAAAGT AAGGGTATCA

AGGCTAAGAT AACCTATGTG GATATCCAAA GTTACTTCAT TGTGCTCAGC

ATAGCTTATC CGTCACTCTC CGAAATTAAG GGGGTGATAG TGCACCGTCT

GGAGACTGTC TCTTACAATA TAGGATCGCA GGAGTGGTAC ACCACAGTAC

CGCGGTATGT GGCAACCCAG GGTTACCTCA TTTCAAATTT CGATGACACA

CCCTGTGCAT TCACTCCGGA GGGCACTATC TGCAGTCAAA ATGCAATATA

CCCGATGAGC CCATTGCTCC AAGAATGTTT CCGTGGGTCA ACCAGGTCAT

GTGCCCGTAC ACTGGTTTTG GGGTCCATAG GGAATAGGTT TATACTATCT

AAGGGGAACC TCATTGGCAA CTGTGCCTCT ATTTGTGCA AGTGCTACAC

CACTGGCTCA ATAATAAGTC AAGACCCTGA CAAGATCCTA ACATATATAG

CTGCAGACCA ATGCCCCGTC GTAGAAGTAG GCGGTGTAAC CATCCAGGTC

GGGAGCAGGG AGTACTCCGA TGCAGTGTAC CTGCATGAAA TAGATCTTGG

CCCACCAATA TCGCTAGAGA AGCTGGATGT GGGGACTAAC CTCTGGAATG

CAGTGACAAA ATTGGAGAAA GCCAAGGATC TACTAGATTC ATCTGATCTG

ATCCTGGAAA ACATCAAGGG TGTTTCAGTC ACGAACACAG GTTACATTOT

AGTCGGAGTC GGGTTGATTG CAGTGGTGGG GATCCTCATT ATTACCTGTT

GTTGTAAGAA GCGCAGGACT GACAACAAAG TCTCCACTAT GGTCTTGAAC

CCGGGTCTTA GACCAGATCT TACTGGTACA TCTAAATCCT ACGTACGGTC

GTTGTAG;
```

or a DNA sequence hybridizable with the aforesaid sequence and encoding part at least of a rinderpest virus antigenic polypeptide or a DNA sequence as aforesaid modified by the deletion or addition of one or more codons or the modification of one or more codons but still encoding a rinderpest virus antigenic polypeptide.

2. The use of DNA according to claim 1 in the preparation of a vaccine for a rinderpest virus.

3. The use of DNA according to claim 1 (a) in constructing a recombinant virus, (b) in producing antigen protein, (c) as a hybridization probe, or (d) in a kit for the diagnosis or detection of rinderpest virus or related viruses.

4. A plasmid or other expression vector incorporating DNA according to claim 1 or a host cell incorporating said plasmid or vector in its expressible genone.

5. A plasmid or vector or cell according to claim 4 and obtainable from FERM BP-1164, FERM BP-1165, FERM BP-1318, or FERM BP-1319.

**Patentansprüche**

1. DNA kodierend für die Gesamtheit oder eines Teiles eines Antigenproteins eines Rinderpestvirus, worin das Antigenprotein ist:
   a) Ein Hämagglutinin-Protein, kodiert durch die Nukleotidsequenz:

```
                    ATGTCCTCCC CAAGAGACAG GGTCAATGCC
TTCTACAAAG ACAACCTCCA ATTTAAGAAC ACTCGAGTGG TTCTTAATAA
AGAGCAGCTC CTGATAGAAA GGCCTTACAT GTTGCTGGCG GTGCTGTTTG
TTATGTTCCT GAGCCTAGTG GGGCTGTTGG CCATTGCAGG TATCAGACTC
CACCGAGCTG CTGTCAACAC AGCAGAGATC AACAGTGGTC TGACGACAAG
CATTGATATT ACCAAGTCTA TTGAGTACCA GGTCAAGGAC GTCTTAACTC
CCCTCTTCAA AATAATTGGA GATGAGGTCG GGCTGAGGAC ACCTCAGAGA
TTCACAGATC TGACTAAATT CATATCAGAC AAGATTAAGT TCCTTAACCC
TGATAAAGAG TACGACTTCA GGGATATTAA CTGGTGCATC AGTCCCCCAG
AGAGAATCAA GATTAATTAT GATCAGTATT GTGCTCACAC AGCTGCTGAG
GAGCTGATAA CTATGCTGGT CAATTCGTCT CTGGCAGGTA CTTCGGTACT
ACCGACATCA TTAGTCAACT TGGGGAGGAG CTGTACCGGG TCCACAACGA
CTAAAGGTCA ATTCTCTAAC ATGTCATTGG CTCTTTCAGG GATATACTCA
GGTCGTGGCT ACAATATTTC ATCCATGATA ACAATCACTG AGAAAGGCAT
GTACGGAAGC ACTTATCTAG TCGGGAAACA TAATCAGGGA GCCAGGAGGC
CAAGCACTGC TTGGCAACGG GATTACCGAG TCTTTGAAGT AGGCATAATT
AGAGAACTAG GACTGGGCAC ACCAGTGTTT CATATGACAA ACTACCTGGA
GCTCCCAAGA CAGCCGGAAT TGGAGATCTG CATGCTAGCT CTAGGAGAGT
TCAAATTAGC TGCCCTCTGC TTAGCTGATA ACTCTGTCGC ACTGCATTAC
GGGGGGTTAA GGGACGACCA CAAGATCAGG TTTGTCAAAC TGGGAGTATG
GCCATCACCA GCCGACTCAG ACACCCTGGC CACTCTTTCA GCAGTAGATC
CGACCTTGGA TGGGCTCTAT ATCACAACTC ATAGGGGAAT CATAGCTGCA
```

13

```
GGGAAGGCCG TATGGGTCGT CCCTGTGACG AGAACAGATG ACCAAAGGAA

AATGGGACAG TGCCGCCGAG AGGCTTGTCG AGAGAAACCA CCACCTTTCT

GTAACAGTAC AGATTGGGAG CCATTAGAGG CCGGCCGTAT ACCGGCATAT

GGAATACTAA CTATCAGGCT GGGGCTGGCT GATAAGCTGA AATTGACCAT

AATTTCAGAA TTTGGTCCCT TGATCACACA TGACTCAGGG ATGGACTTAT

ACACCCCACT TGACGGTAAT GAGTACTGGC TGACTATTCC TCCATTGCAG

AATTCAGCTT TAGGAACGGT GAACACCCTA GTTTTAGAGC CCAGTCTCAA

AATTAGTCCT AACATCCTTA CTCTCCCCAT CAGGTCGGGG GGAGGTGACT

GTTACACTCC CACTTACCTG TCAGACCTGG CCGATGATGA TGTTAAACTG

AGCTCCAATC TTGTAATCCT CCCGAGTAGA AACCTCCAAT ATGTGTCAGC

AACCTACGAC ACCTCTAGAG TTGAGCATGC CATTGTATAC TATATCTATA

GCGCCGGGCG ACTATCATCG TATTACTACC CTGTTAAGTT GCCCATAAAG

GGAGATCCTG TCAGCCTGCA GATAGGATGC TTCCCTTGGG GCCTCAAGCT

ATGGTGCCAT CATTTCTGCT CTGTTATAGA TTCAGGAACT CGCAAGCAGG

TCACCCATAC AGGGGCAGTA GGGATTGAGA TCACTTGCAA TAGCAGATAG;
```

oder eine DNA-Sequenz, welche mit der zuvor genannten Sequenz hybridisierbar ist und wenigstens einen Teil eines antigenen Polypeptides eines Rinderpestvirus kodiert oder eine DNA-Sequenz wie zuvor genannt, modifiziert durch die Deletion oder Addition eines oder mehrerer Codons oder die Modifikation eines oder mehrerer Codons, jedoch noch ein antigenes Polypeptid eines Rinderpestvirus kodierend oder

b) ein Fusionsprotein, kodiert durch die Nukleotidsequenz:

```
          ATGG GGATCTTATT TGCTGCCCTG CTAGCAATGA

CTAACCCACA CTTAGCTACT GGCCAGATCC ACTGGGGCAA CCTTTCAAAA

ATTGGACTCG TAGGGACAGG TAGTGCCAGT TATAAGGTGA TGACCCAATC

GAGTCACCAG TCACTAGTTA TAAAGCTGAT GCCCAATATC ACTGCTATAG

ACAATTGCAC CAAAACAGAG ATTATGGAGT ACAAGAGGCT GTTGGGGACA

GTGCTTAAGC CTATCAGGGA AGCTCTTAAC GCTATTACTA GAACATAAA

ACCAATCCAG AGTTCCACTA CCAGTAGGAG ACACAAGAGG TTCGCGGGAG

TCGTTTTGGC TGGAGCCGCT CTCGGGGTTG CAACCGCAGC CCAGATTACA

GCAGGGATTG CTCTTCATCA GACAATGATG AATTCCCAAG CTATCGAAAG

TCTTAAGGCA AGTCTGGAAA CAACCAATCA AGCAATTGAG GAAATACGAC

AAGCGGGTCA AGAAATGGTC CTAGCGGTTC AGGGTGTCCA AGATTACATC

AACAATGAAC TGGTGCCTGC GATGGGCCAG CTATCGTGTG AAATTGTGGG

TCAAAAGCTA GGGCTGAAAC TGCTTAGATA TTACACTGAA ATCTTGTCAT
```

```
TATTTGGACC CAGCCTCAGG GACCCGGTCT CGGCTGAGCT TTCTATTCAG
GCCTTAAGTT ACGCCTTGGG TGGGGACATT AATAAAATAC TAGAAAAGCT
TGGGTATAGC CCCACTGATC TCCTTGCTAT ACTGGAAAGT AAGGGTATCA
AGGCTAAGAT AACCTATGTG GATATCCAAA GTTACTTCAT TGTGCTCAGC
ATAGCTTATC CGTCACTCTC CGAAATTAAG GGGGTGATAG TGCACCGTCT
GGAGACTGTC TCTTACAATA TAGGATCGCA GGAGTGGTAC ACCACAGTAC
CGCGGTATGT GGCAACCCAG GGTTACCTCA TTTCAAATTT CGATGACACA
CCCTGTGCAT TCACTCCGGA GGGCACTATC TGCAGTCAAA ATGCAATATA
CCCGATGAGC CCATTGCTCC AAGAATGTTT CCGTGGGTCA ACCAGGTCAT
GTGCCCGTAC ACTGGTTTTG GGGTCCATAG GGAATAGGTT TATACTATCT
AAGGGGAACC TCATTGGCAA CTGTGCCTCT ATTTTGTGCA AGTGCTACAC
CACTGGCTCA ATAATAAGTC AAGACCCTGA CAAGATCCTA ACATATATAG
CTGCAGACCA ATGCCCCGTC GTAGAAGTAG GCGGTGTAAC CATCCAGGTC
GGGAGCAGGG AGTACTCCGA TGCAGTGTAC CTGCATGAAA TAGATCTTGG
CCCACCAATA TCGCTAGAGA AGCTGGATGT GGGGACTAAC CTCTGGAATG
CAGTGACAAA ATTGGAGAAA GCCAAGGATC TACTAGATTC ATCTGATCTG
ATCCTGGAAA ACATCAAGGG TGTTTCAGTC ACGAACACAG GTTACATTCT
AGTCGGAGTC GGGTTGATTG CAGTGGTGGG GATCCTCATT ATTACCTGTT
GTTGTAAGAA GCGCAGGACT GACAACAAAG TCTCCACTAT GGTCTTGAAC
CCGGGTCTTA GACCAGATCT TACTGGTACA TCTAAATCCT ACGTACGGTC
GTTGTAG;
```

oder eine DNA-Sequenz, welche mit der zuvor genannten Sequenz hybridisierbar ist und wenigstens einen Teil eines antigenen Polypeptids eines Rinderpestvirus kodiert oder eine DNA-Sequenz wie zuvor genannt, modifiziert durch die Deletion oder Addition eines oder mehrerer Codons oder die Modifikation eines oder mehrerer Codons, jedoch noch ein antigenes Polypeptid eines Rinderpestvirus kodierend.

2. Die Verwendung von DNA gemäß Anspruch 1 in der Herstellung eines Impfstoffs für ein Rinderpestvirus.

3. Die Verwendung von DNA nach Anspruch 1 (a) zum Konstruieren eines rekombinanten Virus, (b) zum Herstellen eines Antigenproteins, (c) als eine Hybridisierungssonde oder (d) in einem Kit zur Diagnose oder Bestimmung des Rinderpestvirus oder verwandten Viren.

4. Ein Plasmid oder anderer Expressionsvektor, eine DNA gemäß Anspruch 1 inkorporierend oder eine Wirtszelle, das Plasmid oder den Vektor in seinem exprimierbaren Genom inkorporierend.

5. Ein Plasmid oder Vektor oder Zelle nach Anspruch 4 und welche erhältlich sind aus FERM BP-1164, FERM BP-1165, FERM BP-1318, oder FERM BP-1319.

**Revendications**

1. De l'ADN codant pour tout ou pärtie d'une protéine d'antigène d'un virus de rinderpest où ladite protéine d'antigène est:
   a) Une protéine d'hémagglutinine pour laquelle code la séquence de nucléotides:

```
                ATGTCCTCCC CAAGAGACAG GGTCAATGCC
TTCTACAAAG ACAACCTCCA ATTTAAGAAC ACTCGAGTGG TTCTTAATAA
AGAGCAGCTC CTGATAGAAA GGCCTTACAT GTTGCTGGCG GTGCTGTTTG
TTATGTTCCT GAGCCTAGTG GGGCTGTTGG CCATTGCAGG TATCAGACTC
CACCGAGCTG CTGTCAACAC AGCAGAGATC AACAGTGGTC TGACGACAAG
CATTGATATT ACCAAGTCTA TTGAGTACCA GGTCAAGGAC GTCTTAACTC
CCCTCTTCAA AATAATTGGA GATGAGGTCG GGCTGAGGAC ACCTCAGAGA
TTCACAGATC TGACTAAATT CATATCAGAC AAGATTAAGT CCTTAACCCC
TGATAAAGAG TACGACTTCA GGGATATTAA CTGGTGCATC AGTCCCCCAG
AGAGAATCAA GATTAATTAT GATCAGTATT GTGCTCACAC AGCTGCTGAG
GAGCTGATAA CTATGCTGGT CAATTCGTCT CTGGCAGGTA CTTCGGTACT
ACCGACATCA TTAGTCAACT TGGGGAGGAG CTGTACCGGG TCCACAACGA
CTAAAGGTCA ATTCTCTAAC ATGTCATTGG CTCTTTCAGG GATATACTCA
GGTCGTGGCT ACAATATTTC ATCCATGATA ACAATCACTG AGAAAGGCAT
GTACGGAAGC ACTTATCTAG TCGGGAAACA TAATCAGGGA GCCAGGAGGC
CAAGCACTGC TTGGCAACGG GATTACCGAG TCTTTGAAGT AGGCATAATT
AGAGAACTAG GACTGGGCAC ACCAGTGTTT CATATGACAA ACTACCTGGA
GCTCCCAAGA CAGCCGGAAT TGGAGATCTG CATGCTAGCT CTAGGAGAGT
TCAAATTAGC TGCCCTCTGC TTAGCTGATA ACTCTGTCGC ACTGCATTAC
GGGGGGGTTAA GGGACGACCA CAAGATCAGG TTTGTCAAAC TGGGAGTATG
GCCATCACCA GCCGACTCAG ACACCCTGGC CACTCTTTCA GCAGTAGATC
CGACCTTGGA TGGGCTCTAT ATCACAACTC ATAGGGGAAT CATAGCTGCA
```

```
GGGAAGGCCG TATGGGTCGT CCCTGTGACG AGAACAGATG ACCAAAGGAA
AATGGGACAG TGCCGCCGAG AGGCTTGTCG AGAGAAACCA CCACCTTTCT
GTAACAGTAC AGATTGGGAG CCATTAGAGG CCGGCCGTAT ACCGGCATAT
GGAATACTAA CTATCAGGCT GGGGCTGGCT GATAAGCTGA AATTGACCAT
AATTTCAGAA TTTGGTCCCT TGATCACACA TGACTCAGGG ATGGACTTAT
ACACCCCACT TGACGGTAAT GAGTACTGGC TGACTATTCC TCCATTGCAG
AATTCAGCTT TAGGAACGGT GAACACCCTA GTTTTAGAGC CCAGTCTCAA
AATTAGTCCT AACATCCTTA CTCTCCCCAT CAGGTCGGGG GGAGGTGACT
GTTACACTCC CACTTACCTG TCAGACCTGG CCGATGATGA TGTTAAACTG
AGCTCCAATC TTGTAATCCT CCCGAGTAGA AACCTCCAAT ATGTGTCAGC
AACCTACGAC ACCTCTAGAG TTGAGCATGC CATTGTATAC TATATCTATA
GCGCCGGGCG ACTATCATCG TATTACTACC CTGTTAAGTT GCCCATAAAG

GGAGATCCTG TCAGCCTGCA GATAGGATGC TTCCCTTGGG GCCTCAAGCT
ATGGTGCCAT CATTTCTGCT CTGTTATAGA TTCAGGAACT CGCAAGCAGG
TCACCCATAC AGGGGCAGTA GGGATTGAGA TCACTTGCAA TAGCAGATAG;
```

ou une séquence d'ADN hybridable avec la séquence susmentionnée et codant pour une partie au moins d'un polypeptide antigénique du virus de rinderpest ou une séquence d'ADN comme susmentionnée modifiée par la délétion ou l'addition d'un codon ou plus ou la modification d'un codon ou plus mais codant encore pour un polypeptide antigénique du virus de rinderpest ou
b) une protéine fusionnée pour laquelle code la séquence de nucléotides:

```
            ATGG GGATCTTATT TGCTGCCCTG CTAGCAATGA
CTAACCCACA CTTAGCTACT GGCCAGATCC ACTGGGGCAA CCTTTCAAAA
ATTGGACTCG TAGGGACAGG TAGTGCCAGT TATAAGGTGA TGACCCAATC
GAGTCACCAG TCACTAGTTA TAAAGCTGAT GCCCAATATC ACTGCTATAG
ACAATTGCAC CAAAACAGAG ATTATGGAGT ACAAGAGGCT GTTGGGGACA
GTGCTTAAGC CTATCAGGGA AGCTCTTAAC GCTATTACTA AGAACATAAA
ACCAATCCAG AGTTCCACTA CCAGTAGGAG ACACAAGAGG TTCGCGGGAG
TCGTTTTGGC TGGAGCCGCT CTCGGGGTTG CAACCGCAGC CCAGATTACA
GCAGGGATTG CTCTTCATCA GACAATGATG AATTCCCAAG CTATCGAAAG
TCTTAAGGCA AGTCTGGAAA CAACCAATCA AGCAATTGAG GAAATACGAC
AAGCGGGTCA AGAAATGGTC CTAGCGGTTC AGGGTGTCCA AGATTACATC
```

17

```
AACAATGAAC TGGTGCCTGC GATGGGCCAG CTATCGTGTG AAATTGTGGG

TCAAAAGCTA GGGCTGAAAC TGCTTAGATA TTACACTGAA ATCTTGTCAT

TATTTGGACC CAGCCTCAGG GACCCGGTCT CGGCTGAGCT TTCTATTCAG

GCCTTAAGTT ACGCCTTGGG TGGGGACATT AATAAAATAC TAGAAAAGCT

TGGGTATAGC CCCACTGATC TCCTTGCTAT ACTGGAAAGT AAGGGTATCA

AGGCTAAGAT AACCTATGTG GATATCCAAA GTTACTTCAT TGTGCTCAGC

ATAGCTTATC CGTCACTCTC CGAAATTAAG GGGGTGATAG TGCACCGTCT

GGAGACTGTC TCTTACAATA TAGGATCGCA GGAGTGGTAC ACCACAGTAC

CGCGGTATGT GGCAACCCAG GGTTACCTCA TTTCAAATTT CGATGACACA

CCCTGTGCAT TCACTCCGGA GGGCACTATC TGCAGTCAAA ATGCAATATA

CCCGATGAGC CCATTGCTCC AAGAATGTTT CCGTGGGTCA ACCAGGTCAT

GTGCCCGTAC ACTGGTTTTG GGGTCCATAG GGAATAGGTT TATACTATCT

AAGGGGAACC TCATTGGCAA CTGTGCCTCT ATTTTGTGCA AGTGCTACAC

CACTGGCTCA ATAATAAGTC AAGACCCTGA CAAGATCCTA ACATATATAG

CTGCAGACCA ATGCCCCGTC GTAGAAGTAG GCGGTGTAAC CATCCAGGTC

GGGAGCAGGG AGTACTCCGA TGCAGTGTAC CTGCATGAAA TAGATCTTGG

CCCACCAATA TCGCTAGAGA AGCTGGATGT GGGGACTAAC CTCTGGAATG

CAGTGACAAA ATTGGAGAAA GCCAAGGATC TACTAGATTC ATCTGATCTG

ATCCTGGAAA ACATCAAGGG TGTTTCAGTC ACGAACACAG GTTACATTOT

AGTCGGAGTC GGGTTGATTG CAGTGGTGGG GATCCTCATT ATTACCTGTT

GTTGTAAGAA GCGCAGGACT GACAACAAAG TCTCCACTAT GGTCTTGAAC

CCGGGTCTTA GACCAGATCT TACTGGTACA TCTAAATCCT ACGTACGGTC

GTTGTAG;
```

ou une séquence d'ADN hybridable avec la séquence susmentionnée et codant en partie au moins pour un polypeptide antigénique de virus de rinderpest ou une séquence d'ADN comme susmentionnée modifiée par la délétion ou l'addition d'un codon ou plus ou la modification d'un codon ou plus mais codant encore pour un polypeptide antigénique du virus de rinderpest.

2. L'utilisation d'ADN selon la revendication 1 dans la préparation d'un vaccin pour un virus de rinderpest.

3. L'utilisation d'ADN selon la revendication 1 (a) dans la construction d'un virus recombinant, (b) dans la production d'une protéine d'antigène, (c) comme une sonde d'hybridation, ou (d) dans un ensemble pour le diagnostic ou la détection du virus de rinderpest ou de virus en rapport.

4. Un plasmide ou autre vecteur d'expression incorporant l'ADN selon la revendication 1 ou une cellule hôte incorporant ledit plasmide ou vecteur dans son génôme expressible.

5. Un plasmide ou vecteur ou cellule selon la revendication 4 et pouvant être obtenu de FERM BP-1164, FERM BP-1165, FERM BP-1318, ou FERM BP-1319.

# Fig. 1

Fig. 2

RV-H-2

RV-F-2

# Fig. 3-1

```
                            ┌─►Start
            20              │               40                    60                      80
AGGATGCAAG ATCATCCACC ATGTCCTCCC CAAGAGACAG GGTCAATGCC TTCTACAAAG ACAACCTCCA ATTTAAGAAC


   XhoI           100                    120                     140                     160
ACTCGAGTGG TTCTTAATAA AGAGCAGCTC CTGATAGAAA GGCCTTACAT GTTGCTGGCG GTGCTGTTTG TTATGTTCCT


                 180                    200                     220                     240
GAGCCTAGTG GGGCTGTTGG CCATTGCAGG TATCAGACTC CACCGAGCTG CTGTCAACAC AGCAGAGATC AACAGTGGTC


                 260                    280                     300                     320
TGACGACAAG CATTGATATT ACCAAGTCTA TTGAGTACCA GGTCAAGGAC GTCTTAACTC CCCTCTTCAA AATAATTGGA


                 340              BglII  360                     380                     400
GATGAGGTCG GGCTGAGGAC ACCTCAGAGA TTCACAGATC TGACTAAATT CATATCAGAC AAGATTAAGT TCCTTAACCC


                 420                    440                     460                     480
TGATAAAGAG TACGACTTCA GGGATATTAA CTGGTGCATC AGTCCCCCAG AGAGAATCAA GATTAATTAT GATCAGTATT


          PvuII  500                    520                     540                     560
GTGCTCACAC AGCTGCTGAG GAGCTGATAA CTATGCTGGT CAATTCGTCT CTGGCAGGTA CTTCGGTACT ACCGACATCA
```

# Fig. 3-2

```
          580                 600                 620                 640
TTAGTCAACT TGGGGAGGAG CTGTACCGGG TCCACAACGA CTAAAGGTCA ATTCTCTAAC ATGTCATTGG CTCTTTCAGG


          660                 680                 700                 720
GATATACTCA GGTCGTGGCT ACAATATTTC ATCCATGATA ACAATCACTG AGAAAGGCAT GTACGGAAGC ACTTATCTAG


          740                 760                 780                 800
TCGGGAAACA TAATCAGGGA GCCAGGAGGC CAAGCACTGC TTGGCAACGG GATTACCGAG TCTTTGAAGT AGGCATAATT


          820                 840          SacI   860                 BglII  880
AGAGAACTAG GACTGGGCAC ACCAGTGTTT CATATGACAA ACTACCTGGA GCTCCCAAGA CAGCCGGAAT TGGAGATCTG


SphI          900                 920                 940                 960
CATGCTAGCT CTAGGAGAGT TCAAATTAGC TGCCCTCTGC TTAGCTGATA ACTCTGTCGC ACTGCATTAC GGGGGGTTAA


          980                 1000                1020                1040
GGGACGACCA CAAGATCAGG TTTGTCAAAC TGGGAGTATG GCCATCACCA GCCGACTCAG ACACCCTGGC CACTCTTTCA


          1060                1080                1100 PstI            1120
GCAGTAGATC CGACCTTGGA TGGGCTCTAT ATCACAACTC ATAGGGGAAT CATAGCTGCA GGGAAGGCCG TATGGGTCGT
```

EP 0 257 994 B1

# Fig. 3-3

```
                 1140                    1160                    1180                    1200
CCCTGTGACG AGAACAGATG ACCAAAGGAA AATGGGACAG TGCCGCCGAG AGGCTTGTCG AGAGAAACCA CCACCTTTCT


                 1220                    1240                    1260                    1280
GTAACAGTAC AGATTGGGAG CCATTAGAGG CCGGCCGTAT ACCGGCATAT GGAATACTAA CTATCAGGCT GGGGCTGGCT


                 1300                    1320                    1340                    1360
GATAAGCTGA AATTGACCAT AATTTCAGAA TTTGGTCCCT TGATCACACA TGACTCAGGG ATGGACTTAT ACACCCCACT


                 1380                    1400                    1420                    1440
TGACGGTAAT GAGTACTGGC TGACTATTCC TCCATTGCAG AATTCAGCTT TAGGAACGGT GAACACCCTA GTTTTAGAGC


                 1460                    1480                    1500                    1520
CCAGTCTCAA AATTAGTCCT AACATCCTTA CTCTCCCCAT CAGGTCGGGG GGAGGTGACT GTTACACTCC CACTTACCTG


                 1540          SacI      1560                    1580                    1600
TCAGACCTGG CCGATGATGA TGTTAAACTG AGCTCCAATC TTGTAATCCT CCCGAGTAGA AACCTCCAAT ATGTGTCAGC


         XbaI  1620                      1640                    1660                    1680
AACCTACGAC ACCTCTAGAG TTGAGCATGC CATTGTATAC TATATCTATA GCGCCGGGCG ACTATCATCG TATTACTACC
```

EP 0 257 994 B1

## Fig. 3-4

EP 0 257 994 B1

```
                1700                      1720 PstI            1740                    1760
CTGTTAAGTT GCCCATAAAG GGAGATCCTG TCAGCCTGCA GATAGGATGC TTCCCTTGGG GCCTCAAGCT ATGGTGCCAT

                1780                      1800                 1820                    1840
CATTTCTGCT CTGTTATAGA TTCAGGAACT CGCAAGCAGG TCACCCATAC AGGGGCAGTA GGGATTGAGA TCACTTGCAA

  Stop
                1860              BglII 1880             PvuII 1900                    1920
TAGCAGATAG CAGTGTCTTG GCCCTACAAG ATCTCGGAGA CCGGGACCCC CAACAGCTGT GGGACCAGGC ACCGCGCTGC

                1940
ACCATGCAGA CAGCTTTCAA TATTACCATT ATA(n)
```

## Fig. 4 - 1

```
                 20                    40                    60                    80
AGGGCCAAAG AATCACATCG ACCCAAGCCG CCAGAAAGGA CAACACCATC GCCACCCAAC CAAACTGGAA ACCAGGGAGC


                100                   120                   140                   160
AGCCACCCGC AGGGCCCAAA GCCCGGGCCC ACCCCACGAG CAGGGCGGAA CCAAGGGGCA GGCCCGCCCC ATGCACAGCA


                180                   200                   220                   240
ACAGAACACC CCCAGATCCC CCACCAAAAG CCCCCCCGAC ACCGGGCAGA ACCCGAGGGC ACCCAGGAGA ATCCCCCGAC


                260                   280                   300                   320
CCCAGACCCC CAACAGCAGC ATCCCCCACC CACCCCCCCC GTTCACTCCT CCCGGAACTT CCACTCCCAC CCCCTCCCGA


                340                   360                   380                   400
GGAACAGAGG GCCCCATCCC CCCACTCCTC CCCGCCCCGG TCTCCAGCCC GAGACAACCC CCACACCCCA ACACGGAAGG


                420                   440                   460                   480
TCTCGGTCGC TCAGCGCCGC CGAAGATCCA GCCAGAAAGA GCACCAAAGG CAGACCACCG GGCCCTGTGG TGGACCCCGC


                500                   520                   540                   560
CAACCGGGAG CCCCAGACCC CCAAATCCAT CCGGGCATCA AATCACGGCA GAGCAACCAA GGCACTGCTC AACAAACATG
```

EP 0 257 994 B1

## Fig. 4-2

Start

CCCAGACCAC CTACAAAGGA CCTAGCATGG GGATCTTATT TGCTGCCCTG CTAGCAATGA CTAACCCACA CTTAGCTACT

GGCCAGATCC ACTGGGGCAA CCTTTCAAAA ATTGGAGTCG TAGGGACAGG TAGTGCCAGT TATAAGGTGA TGACCCAATC

GAGTCACCAG TCACTAGTTA TAAAGCTGAT GCCCAATATC ACTGCTATAG ACAATTGCAC CAAAACAGAG ATTATGGAGT

ACAAGAGGCT GTTGGGGACA GTGCTTAAGC CTATCAGGGA AGCTCTTAAC GCTATTACTA AGAACATAAA ACCAATCCAG

AGTTCCACTA CCAGTAGGAG ACACAAGAGG TTCGCGGGAG TCGTTTTGGC TGGAGCCGCT CTCGGGGTTG CAACCGCAGC

CCAGATTACA GCAGGGATTG CTCTTCATCA GTCAATGATG AATTCCCAAG CTATCGAAAG TCTTAAGGCA AGTCTGGAAA

CAACCAATCA AGCAATTGAG GAAATACGAC AAGCGGGTCA AGAAATGGTC CTAGCGGTTC AGGGTGTCCA AGATTACATC

EP 0 257 994 B1

# Fig. 4-3

EP 0 257 994 B1

```
                1140                    1160                    1180                    1200
AACAATGAAC TGGTGCCTGC GATGGGCCAG CTATCGTGTG AAATTGTGGG TCAAAAGCTA GGGCTGAAAC TGCTTAGATA


                1220                    1240                    1260                    1280
TTACACTGAA ATCTTGTCAT TATTTGGACC CAGCCTCAGG GACCCGGTCT CGGCTGAGCT TTCTATTCAG GCCTTAAGTT


                1300                    1320                    1340                    1360
ACGCCTTGGG TGGGGACATT AATAAAATAC TAGAAAAGCT TGGGTATAGC GGGAGTGATC TCCTTGCTAT ACTGGAAAGT


                1380                    1400                    1420                    1440
AAGGGTATCA AGGCTAAGAT AACCTATGTG GATATCGAAA GTTACTTCAT TGTGCTCAGC ATAGCTTATC CGTCACTCTC


                1460                    1480                    1500                    1520
CGAAATTAAG GGGGTGATAG TGCACCGTCT GGAGAGTGTC TCTTACAATA TAGGATCGCA GGAGTGGTAC ACCACAGTAC


                1540                    1560                    1580                    1600
CGCGGTATGT GGCAACCCAG GGTTACCTCA TTTCAAATTT CGATGACACA CCCTGTGCAT TCACTCCGGA GGGCACTATC


                1620                    1640                    1660                    1680
TGCAGTCAAA ATGCAATATA CCCGATGAGC CCATTGCTCC AAGAATGTTT CCGTGGGTCA ACCAGGTCAT GTGCCCGTAC
```

# Fig. 4-4

```
                  1700                1720                1740                1760
ACTGGTTTTG GGGTCCATAG GGAATAGGTT TATACTATCT AAGGGGAACC TCATTGGCAA CTGTGCCTCT ATTTTGTGCA


                  1780                1800                1820                1840
AGTGCTACAC CACTGGCTCA ATAATAAGTC AAGACCCTGA CAAGATCCTA ACATATATAG CTGCAGACCA ATGCCCCGTC


                  1860                1880                1900                1920
GTAGAAGTAG GCGGTGTAAC CATCCAGGTC GGGAGCAGGG AGTACTCCGA TGCAGTGTAC CTGCATGAAA TAGATCTTGG


                  1940                1960                1980                2000
CCCACCAATA TCGCTAGAGA AGCTGGATGT GGGGACTAAC CTCTGGAATG CAGTGACAAA ATTGGAGAAA GCCAAGGATC


                  2020                2040                2060                2080
TACTAGATTC ATCTGATCTG ATCCTGGAAA ACATCAAGGG TGTTTCAGTC ACGAACACAG GTTACATTCT AGTCGGAGTC


                  2100                2120                2140                2160
GGGTTGATTG CAGTGGTGGG GATCCTCATT ATTACCTGTT GTTGTAAGAA GCGCAGGACT GACAACAAAG TCTCCACTAT
                                                                              ← ⌐ Stop
                  2180                2200                2220       ⌐        2240
GGTCTTGAAC CCGGGTCTTA GACCAGATCT TACTGGTACA TCTAAATCCT ACGTACGGTC GTTGTAGCAG GTGTGTCTCC
```

## Fig. 4-5

```
              2260                    2280                   2300                  2320
CATGTGATCA ATGTCCCCGA ATACCTTATC AAACCTTACA ATGCTTTGTC CTCCTCGCAG CCAGCTAGTC GCTATCCTCA

              2340
GCAGGCACCA TGCTCGATCG CTCATTAATT GCTACAAAG
```